(19)

Europäisches
Patentamt
European
Patent Office
Office européen
des brevets

(11)    **EP 3 320 856 A1**

(12)    **EUROPEAN PATENT APPLICATION**

(43) Date of publication:
**16.05.2018 Bulletin 2018/20**

(51) Int Cl.:
**A61B 17/10** (2006.01)      **A61B 17/128** (2006.01)
**A61B 17/12** (2006.01)      A61B 17/00 (2006.01)
A61B 17/29 (2006.01)      A61B 90/00 (2016.01)

(21) Application number: **16198798.7**

(22) Date of filing: **15.11.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **11.11.2016   KR 20160150168**

(71) Applicant: BNR Co., Ltd.
**Incheon (KR)**

(72) Inventors:
• **Bae, Gyeongcheol**
  **Gangnam-gu, Seoul (KR)**

• **Min, Sungki**
  **Dongdaemun-gu, Seoul (KR)**
• **Kim, Gichang**
  **Yeongdeungpo-gu, Seoul (KR)**
• **Jeon, Bonggwan**
  **Bucheon-si, Gyeonggi-do (KR)**
• **Kim, Hoe Chan**
  **Namdong-gu, Incheon (KR)**
• **Bae, Sujin**
  **Gangnam-gu, Seoul (KR)**
• **Hong, Junghwa**
  **Seongbuk-gu, Seoul (KR)**

(74) Representative: **V.O.**
**P.O. Box 87930**
**Carnegieplein 5**
**2508 DH Den Haag (NL)**

(54)    **SURGICAL CLIP APPLIER**

(57)    Disclosed is a surgical clip applier. The surgical clip applier includes a power cutoff device for cutting off power when a high load is applied to, for example, a trigger, jaws, or a power system.

Fig. 8

**Description**

BACKGROUND OF THE INVENTION

Field of the Invention

The present invention relates to a surgical clip applier.

Description of the Related Art

During various kinds of surgical operations, in order to prevent excessive bleeding of patients and to reduce the risks of other complications, blood vessels and the like need to be ligated prior to being cut. There are various technologies for ligating blood vessels and the like. One of them serves to ligate the periphery of a blood vessel using sutures in order to close the blood vessel. In another, a doctor positions a clip, having a pair of legs, around a blood vessel and presses the legs of the clip, as illustrated in the prior art patent document cited below. Such a clip may be applied to, for example, a blood vessel via a surgical clip applier.

Specifically, the surgical clip applier closes the clip while each of a pair of jaws is moved closer to the other when a trigger is pulled in the state in which the clip is located between the jaws. However, the surgical clip applier according to the prior art has a problem in that a high load is applied to, for example, the trigger, the jaws, and a power system that interconnects the trigger and the jaws when the trigger is pulled in the state in which an object that has a predetermined strength or more, such as a bone, is located between the jaws, thus causing the breakage thereof.

[Prior Art Document]

[Patent Document]

(Patent Document 1) KR10-1298521 B1

SUMMARY OF THE INVENTION

Therefore, the present invention has been made in view of the above problems, and it is an object of the present invention to provide a surgical clip applier having a power cutoff device that cuts off power when a high load is applied to, for example, a trigger, jaws, or a power system.

In accordance with an aspect of the present invention, to accomplish the above and other objects, there is provided a surgical clip applier including a handle unit coupled to one side of a case so as to be rotatable about a main shaft, a power transmission unit connected to the handle unit for transmitting power to a pair of jaws at a predetermined position thereof so as to move the jaws closer to each other when the handle unit is rotated, and a power cutoff device provided between the handle unit and the power transmission unit, wherein the power cutoff device includes a first connection member coupled to the handle unit so as to be rotated along with the handle unit about the main shaft, a second connection member coupled to the power transmission unit so as to be rotatable about a rotating shaft, which is formed on the power transmission unit, and a pressure piece for pressing the second connection member so that one surface of the second connection member is brought into contact with and is coupled with one surface of the first connection member, wherein, in a state in which the surface of the first connection member is brought into contact with and is coupled with the surface of the second connection member, when the first connection member is rotated about the main shaft, the second connection member is rotated, along with the first connection member, about the main shaft, and wherein, when a predetermined load or more is applied to the predetermined position on the power transmission unit, the second connection member is rotated about the rotating shaft, and coupling between the first connection member and the second connection member is released.

In the surgical clip applier according to the aspect of the present invention, the surface of the first connection member may be provided with a first convex-concave portion in which convex portions and concave portions are successively formed, the surface of the second connection member may be provided with a second convex-concave portion in which convex portions and concave portions are successively formed, and the first convex-concave portion and the second convex-concave portion may be engaged with and coupled to each other.

In the surgical clip applier according to the aspect of the present invention, the first convex-concave portion may be provided on the surface of the first connection member that extends and has a predetermined curvature, and the second convex-concave portion may be provided on the surface of the second connection member that extends and has a predetermined curvature, so as to correspond to the first convex-concave portion.

In the surgical clip applier according to the aspect of the present invention, the first connection member may have a cylindrical shape so that the first convex-concave portion is formed in a predetermined section of an outer circumferential surface thereof, and the second connection member may extend to have an arc shape so that the second convex-concave portion is formed in a predetermined section of a side surface thereof, which faces the first convex-concave portion.

In the surgical clip applier according to the aspect of the present invention, the first convex-concave portion may be formed in a shape of teeth so as to have a predetermined angle of inclination, the second convex-concave portion may be formed in a shape of teeth so as to have a predetermined angle of inclination, and the predetermined angle of inclination of the first convex-concave portion and the predetermined angle of inclination of the second convex-concave portion may be the same as each other.

In the surgical clip applier according to the aspect of the present invention, the pressure piece may be a spring.

In the surgical clip applier according to the aspect of the present invention, when the predetermined load is defined as "$F_R$", the predetermined angle of inclination of the first convex-concave portion and the second convex-concave portion is defined as "$\theta$", a coefficient of friction between the first convex-concave portion and the second convex-concave portion is defined as "$\mu$", a distance from the main shaft to a position at which the first convex-concave portion and the second convex-concave portion are coupled to each other is defined as "r", a distance from the main shaft to the predetermined position on the power transmission unit is defined as "R", and force with which the pressure piece presses the second connection member is defined as "$F_v$", the following Equation 1 may be satisfied.

$$[Equation\ 1]$$

$$F_V = \cot(\theta + \Phi)\ X\ (R/r)\ X\ F_R,\ here\ \Phi = \tan^{-1}(\mu)$$

In the surgical clip applier according to the aspect of the present invention, the predetermined angle of inclination of the first convex-concave portion and the second convex-concave portion ranges from 60 degrees to 70 degrees.

In the surgical clip applier according to the aspect of the present invention, the power transmission unit may be connected at the predetermined position to a pressure plate, and, when a user rotates the handle unit, the power transmission unit may be rotated about the main shaft, causing the pressure plate to slide.

In the surgical clip applier according to the aspect of the present invention, the pressure plate may be connected to a cam channel, and, when the pressure plate slides, the cam channel may slide toward the jaws, causing a pair of jaw support portions, which support the jaws, to be moved closer to each other within the cam channel, and consequently, causing the jaws to be moved closer to each other.

Features and advantages of the present invention will become clearer from the following detailed description with reference to the accompanying drawings.

The terms or words used in the specification and claims of the present invention should not be interpreted using typical or dictionary limited meanings, and should be constructed as meanings and concepts conforming to the technical sprit of the present invention based on the principle that the inventors can appropriately define the concepts of the terms to explain the present invention in the best manner.

BRIEF DESCRIPTION OF THE DRAWINGS

The above and other objects, features and other advantages of the present invention will be more clearly understood from the following detailed description taken in conjunction with the accompanying drawings, in which:

FIG. 1 is a perspective view of a surgical clip applier according to an embodiment of the present invention;
FIG. 2 is an exploded perspective view of a main body of the surgical clip applier according to the embodiment of the present invention;
FIGS. 3 and 4 are side views illustrating the operating sequence of the surgical clip applier according to the embodiment of the present invention;
FIG. 5 is an exploded perspective view of a shaft and jaws of the surgical clip applier according to the embodiment of the present invention;
FIGS. 6A to 6D are plan views of the shaft and the jaws of the surgical clip applier according to the embodiment of the present invention;
FIGS. 7 and 8 are side views illustrating the operating sequence of a power cutoff device of the surgical clip applier according to the embodiment of the present invention; and
FIGS. 9 and 10 are enlarged views illustrating the power cutoff device of the surgical clip applier according to the embodiment of the present invention.

DETAILED DESCRIPTION OF THE INVENTION

The objects, specific advantages and novel features of the present invention will become clearer from the following detailed description in conjunction with the accompanying drawings and the exemplary embodiments. In the specification, with regard to reference numerals added to constituent elements illustrated in the respective drawings, it is to be noted that the same constituent elements are designated by the same reference numerals even when they are depicted in different drawings. In addition, the terms "first", "second", "one end", "the other end", etc. are used simply to distinguish any one element from other elements, and the elements are not limited by the terms. In the following description of the present invention, a detailed description of known functions incorporated herein will be omitted when it may make the subject matter of the disclosure rather unclear.

Hereinafter, an exemplary embodiment of the present invention will be described in detail with reference to the accompanying drawings.

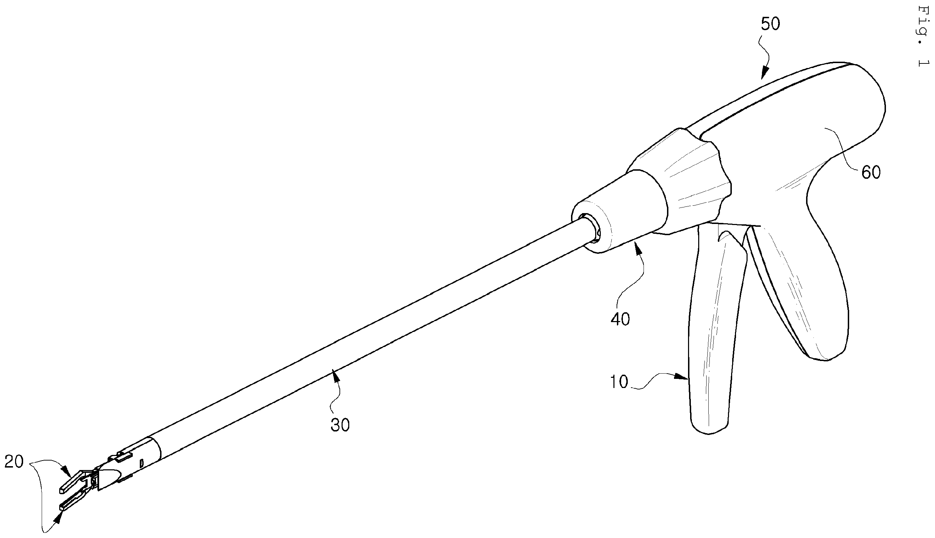

FIG. 1 is a perspective view of a surgical clip applier according to an embodiment of the present invention.

As illustrated in FIG. 1, the surgical clip applier according to the present invention may be used in a trocar in order to mount a clip in a human body. Basically, when a trigger 10 is pulled, jaws 20, forming a pair, are moved closer to each other, causing a clip mounted to the jaws 20 to be ligated to, for example, the blood vessel or the tissues. Thereafter, a new clip may be mounted to the jaws 20. Meanwhile, when a rotating knob 40 provided between a shaft 30 and a main body 50 is rotated, the jaws 20 may be rotated along with the shaft 30.

First, when viewing the external appearance, the surgical clip applier according to the present invention may include the main body 50, the shaft 30 extending from one side of the main body 50, and the jaws 20 arranged at one end of the shaft 30.

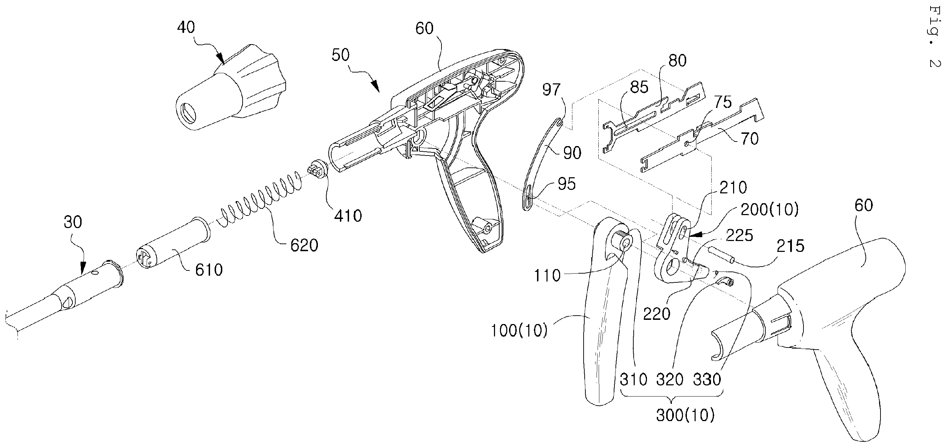

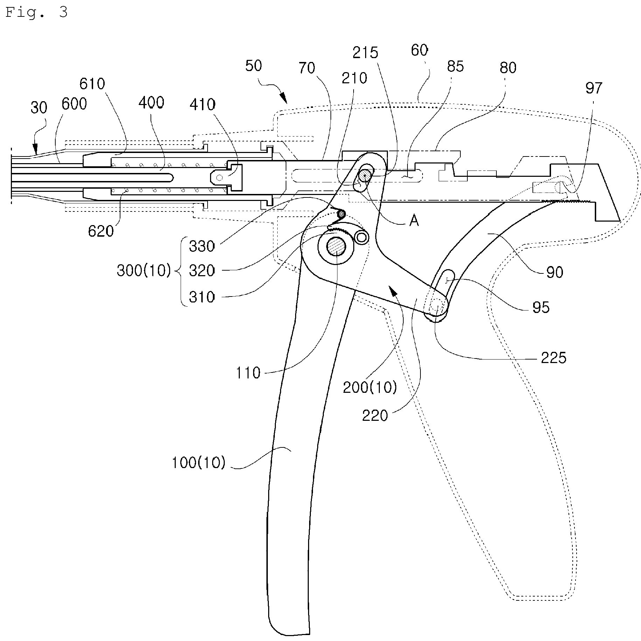

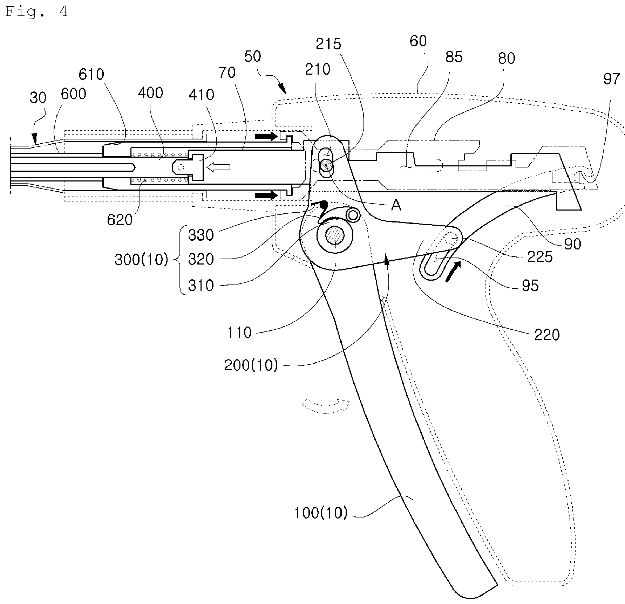

FIG. 2 is an exploded perspective view of the main body of the surgical clip applier according to the embodiment of the present invention, and FIGS. 3 and 4 are side views illustrating the operating sequence of the surgical clip applier according to the embodiment of the present invention.

As illustrated in FIGS. 2 to 4, the main body 50 may include, for example, a case 60, the trigger 10, a pressure plate 70, a transfer plate 80, and a transfer link 90. At this time, the trigger 10 may include a handle unit 100 and a power transmission unit 200. The handle unit 100 is coupled to one side of the case 60 so as to be rotatable about a main shaft 110 and extends outward from the case 60 so as to be exposed. The power transmission unit 200 is connected to the handle unit 100 and transmits power to the jaws 20 through the pressure plate 70 and a cam channel 400 at a predetermined position A (see FIGS. 3 and 4) so as to move the jaws 20 closer to each other when the handle unit 100 is rotated. In addition, the trigger 10 may be provided with a power cutoff device 300 between the handle unit 100 and the power transmission unit 200. A detailed description of the power cutoff device 300 will follow, and the handle unit 100 and the power transmission unit 200 will be described first.

Specifically, the power transmission unit 200 may be provided with a first slot 210 at the predetermined position A, which is located at the opposite side of the handle unit 100 on the basis of the main shaft 110. At this time, the pressure plate 70 may be connected to the power transmission unit 200 at the predetermined position A (i.e. the first slot 210) using a connection pin 215. For example, the connection pin 215 may be inserted into the first slot 210 of the power transmission unit 200 and a connection hole 75 (see FIG. 2) in the pressure plate 70, so as to connect the power transmission unit 200 and the pressure plate 70 to each other. Accordingly, as illustrated in FIGS. 3 and 4, when the user rotates the handle unit 100, the power transmission unit 200 is also rotated about the main shaft 110 so that the pressure plate 70 connected to the power transmission unit 200 may slide toward the shaft 30.

Meanwhile, the power transmission unit 200 may include a pivot body 220, which extends in a given direction (rearward) from the main shaft 110 within the case 60. A protrusion 225 formed on the pivot body 220 may be connected to a second slot 95 formed in one end of the transfer link 90, and an opposite end 97 of the transfer link 90 may be connected to one end of the transfer plate 80. Accordingly, as illustrated in FIGS. 3 and 4, when the user rotates the handle unit 100, the power transmission unit 200 is also rotated about the main shaft 110. Thereby, the protrusion 225 formed on the pivot body 220 of the power transmission unit 200 is moved along the second slot 95 of the transfer link 90 and is caught by the end of the second slot 95. Thereafter, power may be transmitted from the protrusion 225 to the transfer link 90 so that the transfer plate 80 connected to the transfer link 90 may slide away from the shaft 30.

That is, when the user rotates the handle unit 100, the pressure plate 70 slides (forward) toward the shaft 30 as soon as the handle unit 100 is rotated. After the handle unit 100 is rotated at a predetermined angle, the transfer plate 80 may slide (rearward) away from the shaft 30.

In addition, the transfer plate 80 may be provided with a third slot 85 in order to enable the movement of the connection pin 215 that connects the power transmission unit 200 and the pressure plate 70 to each other, which may prevent interference between the pressure plate 70 and the transfer plate 80, both of which slide.

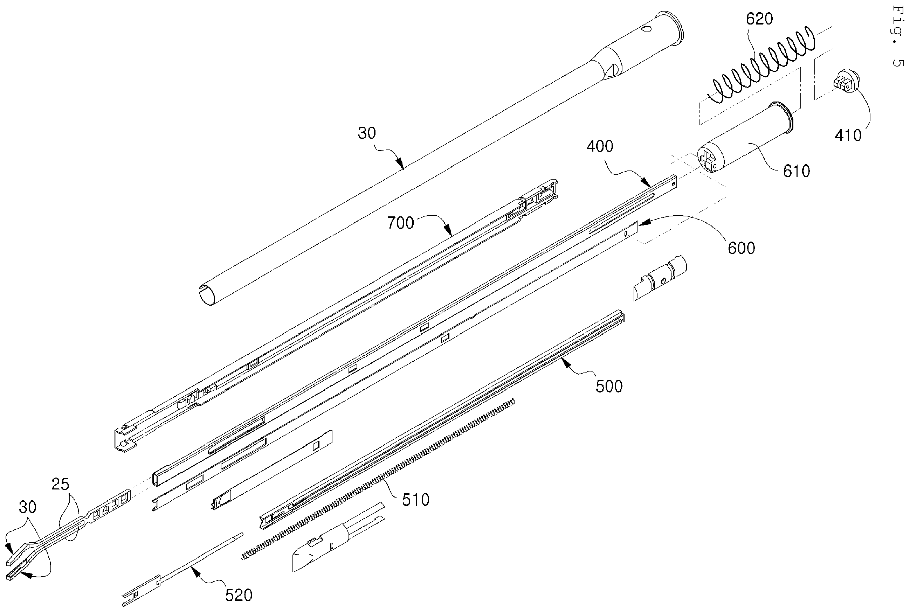

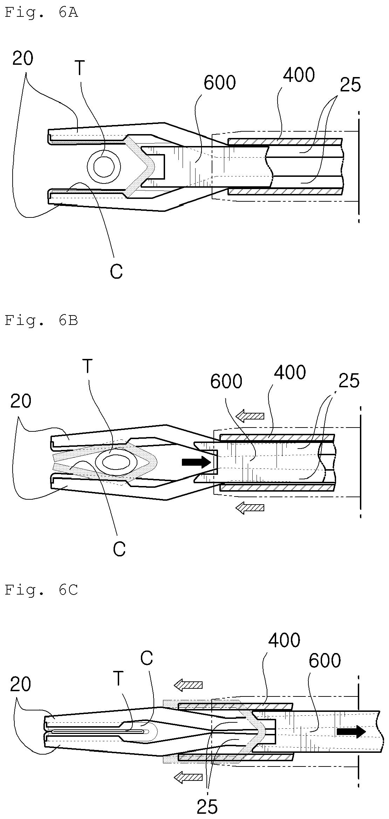

FIG. 5 is an exploded perspective view of the shaft and the jaws of the surgical clip applier according to the embodiment of the present invention, and FIGS. 6A to 6D are plan views of the shaft and the jaws of the surgical clip applier according to the embodiment of the present invention.

As illustrated in FIG. 5, for example, the cam channel 400, a clip track 500, a transfer bar 600, and a support member 700 for supporting, for example, the cam channel 400 may be provided inside the shaft 30.

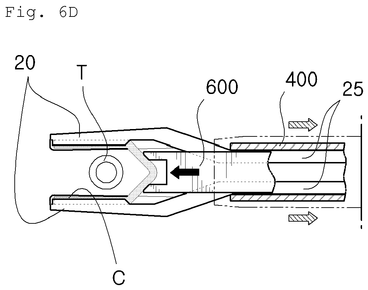

Here, the cam channel 400 may be connected to the above-described pressure plate 70 via a first coupling 410 (see FIG. 3). Accordingly, as illustrated in FIGS. 3 and 4, when the user rotates the handle unit 100, the pressure plate 70 slides (forward) toward the shaft 30, causing the cam channel 40 to slide (forward) toward the jaws 20 within the shaft 30. When the cam channel 400 slides (forward) toward the jaws 20, as illustrated in FIGS. 6A to 6C, a pair of jaw support portions, which support respective jaws 20, are moved closer to each other within the cam channel 400. As a result, the jaws 20 are moved closer to each other so as to close the clip C, thereby allowing the clip to be applied to, for example, the blood vessel or the tissues T. Thereafter, when the user releases the handle unit 100, the cam channel 400 may slide (rearward) away from the jaws 20 by a compression spring 620, which is located between the first coupling 410 and a second coupling 610. In this way, as illustrated in FIG. 6D, the jaw support portions 225, which support respective jaws 20, are moved away from each other, causing the jaws 20 to be moved away from each other. Thereby, a new clip may be mounted to the jaws 20 by the clip track 500 and the transfer bar 600, which will be described below.

The clip track 500 (see FIG. 5) may accommodate a plurality of clips therein, and the clips may be pressed (forward) toward the jaws 20 by a transfer shoe 520 and a transfer spring 510, which are arranged inside the clip track 500.

The transfer bar 600 may be connected to the above-described transfer plate 80 via the second coupling 610 (see FIG. 3). Accordingly, as illustrated in FIGS. 3 and 4, when the user rotates the handle unit 100, the transfer plate 80 slides (rearward) away from the shaft 30, causing the transfer bar 600 to slide (rearward) away from the jaws 20 within the shaft 30. When the transfer bar 600 slides (rearward) away from the jaws 20 within the shaft 30, as illustrated in FIG. 6C, one clip (i.e. the foremost clip among the clips accommodated in the clip track 500) may be caught by the end of the transfer bar 600. Thereafter, when the user releases the handle unit 100, the transfer plate 80 may slide (forward) toward the jaws 20 due to the compression spring 620, which is located between the second coupling 610 and the first coupling 410. As illustrated in FIG. 6D, when the transfer plate 80 slides (forward) toward the jaws 20, the clip caught by the end of the transfer plate 80 (i.e. the foremost clip among the clips accommodated in the clip track 500) is moved (forward) toward the jaws 20, thereby allowing the clip to be finally mounted to the jaws 20.

As described above, when the user rotates the handle unit 100, the pressure plate 70 and the cam channel 400 slide toward the jaws 20, causing the jaws 20 to be moved closer to each other (see FIGS. 6A to 6C). However, when the user forcefully rotates the handle unit 100 in the state in which the jaws 20 cannot be moved closer to each other due to the presence of an object having predetermined strength or more, such as, for example, a bone, between the jaws 20, a high load may be applied to, for example, the jaws 20, the cam channel 40, the pressure plate 70, the power transmission unit 200, and the handle unit 100, which may cause the breakage thereof.

In the case of the surgical clip applier according to the present embodiment, the power cutoff device 300 (see FIGS. 3 and 4) may be provided to cut off the power transmitted from the user when a high load is applied to, for example, the jaws 20, the cam channel 40, the pressure plate 70, the power transmission unit 200, and the handle unit 100, thereby preventing the breakage of, for example, the jaws 20, the cam channel 40, the pressure plate 70, the power transmission unit 200, and the handle unit 100.

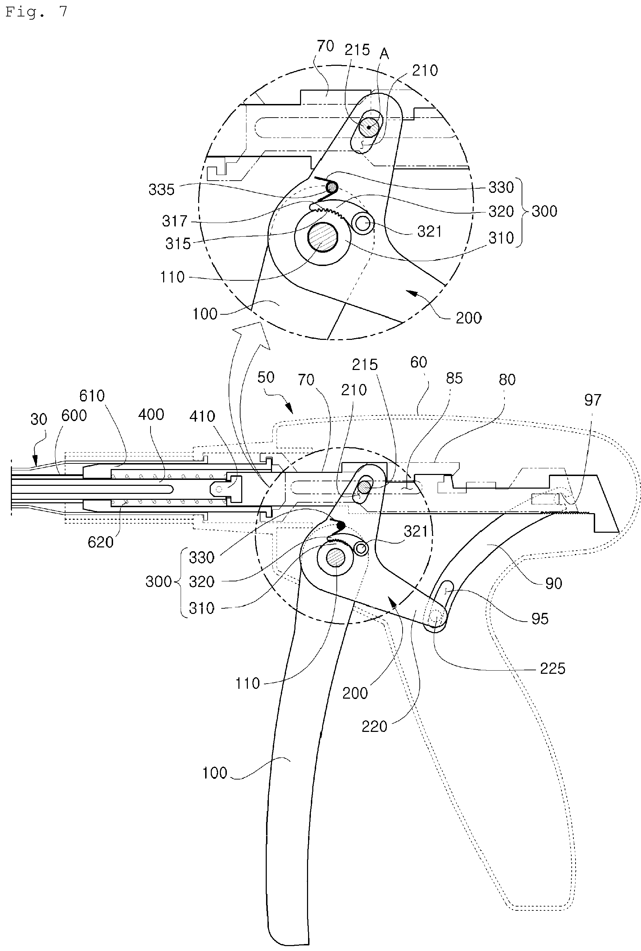

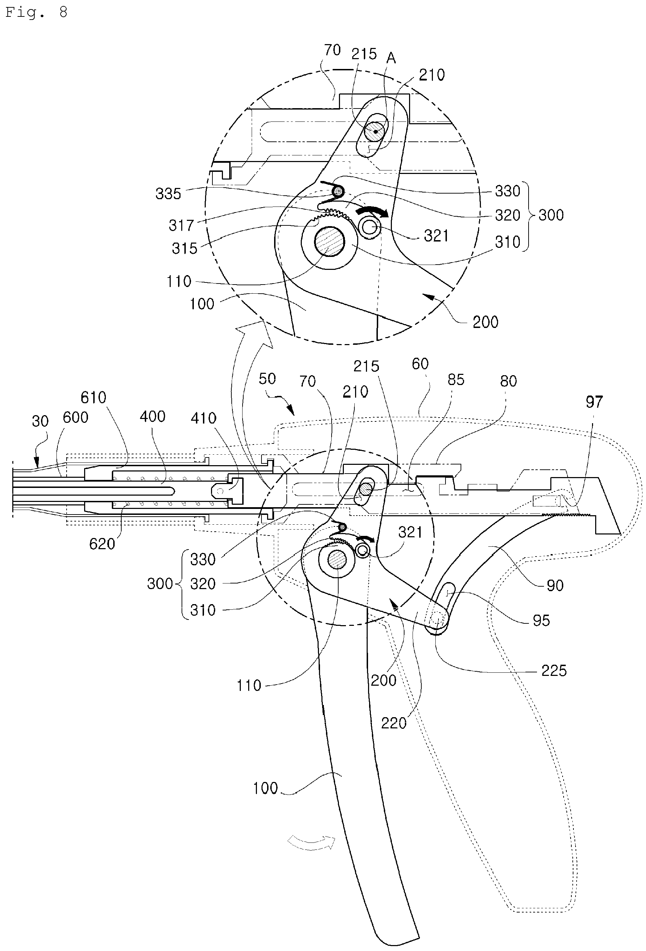

FIGS. 7 and 8 are side views illustrating the operating sequence of the power cutoff device of the surgical clip applier according to the embodiment of the present invention.

As illustrated in FIGS. 7 and 8, the power cutoff device 300 of the surgical clip applier according to the present embodiment may be provided between the handle unit 100 and the power transmission unit 200. Here, the handle unit 100 may be rotated about the main shaft 110 when the user applies force thereto. In addition, although the power transmission unit 200 is basically connected to the handle unit 100 via the power cutoff device 300 so as to be rotated along with the handle unit 100 (see FIGS. 3 and 4), the power transmission unit 200 may be disconnected from the handle unit 100 by the power cutoff device 300 when a high load is applied thereto, and thus may not be rotated along with the handle unit 100 (see FIGS. 7 and 8).

Specifically, the power cutoff device 300 may include a first connection member 310, a second connection member 320, a rotating shaft 321, and a pressure piece 330. Here, the first connection member 310 may be coupled to the handle unit 100 so as to be rotated along with the handle unit 100 about the main shaft 110. The second connection member 320 may be coupled to the power transmission unit 200 so as to be rotated about the rotating shaft 321, which is formed on the power transmission unit 200. The pressure piece 330 serves to press the second connection member 320 toward the first connection member 310 so that one surface of the second connection member 320 is brought into contact with and is coupled with one surface of the first connection member 310. Here, in the state in which the surface of the second connection member 320 is in contact with and is coupled with the surface of the first connection member 310, when the first connection member 310 is rotated about the main shaft 110, the second connection member 320 may be rotated about the main shaft 110 along with the first connection member 310 (see FIGS. 3 and 4). That is, when the first connection member 310 and the second connection member 320 are coupled to each other, the power applied to the handle unit 100 may be transmitted to the power transmission unit 200.

Meanwhile, in order to achieve the coupling of the first connection member 310 and the second connection member 320, as illustrated in FIGS. 7 and 8, the surface of the first connection member 310 may be provided with a first convex-concave portion 315 in which convex portions and concave portions are successively formed, and the surface of the second connection member 320 may be provided with a second convex-concave portion 317 in which convex portions and concave portions are successively formed. At this time, because the first convex-concave portion 315 and the second convex-concave portion 317 are engaged with and coupled to each other, the first connection member 310 and the second connection member 320 may be rotated together about the main shaft 110. More specifically, the first convex-concave portion 315 may be formed on the surface of the first connection member 310, which extends and has a predetermined curvature, and the second convex-concave portion 317 may be formed on the surface of the second connection member 320, which extends and has a predetermined curvature so as to correspond to the first convex-concave portion 315. For example, the first connection member 310 generally has a cylindrical shape so that the main shaft 110 is located at the center thereof, and the first convex-concave portion 315 may be formed in a predetermined section of the outer circumferential surface of the first connection member 310. The second connection member 320 may generally extend to have an arc shape, and the second convex-concave portion 317 may be formed in a predetermined section of the side surface of the second connection member 320, which faces the first convex-concave portion 315. The first convex-concave portion 315 may be formed in the shape of teeth so as to have a predetermined angle of inclination, and the second convex-concave portion 317 may be formed in the shape of teeth so as to have a predetermined angle of inclination. At this time, the predetermined angle of inclination of the first convex-concave portion 315 and the predetermined angle of inclination of the second convex-concave portion 317 may be the same as each other.

Meanwhile, because one end of the second connection member 320 is coupled to the power transmission unit 200 about the rotating shaft 321, the second connection member 320 may be rotated about the rotating shaft 321 relative to the power transmission unit 200. At this time, because the pressure piece 330 presses the second connection member 320 toward the first connection member 310, basically, the second convex-concave portion 317 of the second connection member 320 and the first convex-concave portion 315 of the first connection member 310 are engaged with and coupled to each other. Therefore, when the user rotates the handle unit 100, the power transmission unit 200 is also rotated via the coupling of the first convex-concave portion 315 and the second convex-concave portion 317. Thereby, the pressure plate 70 and the cam channel 400 slide (forward) toward the jaws 20 (see FIGS. 3 and 4), causing the jaws 20 to be moved closer to each other so as to ligate the clip (see FIGS. 6A to 6C).

However, when the user forcefully rotates the handle unit 100 in the state in which the jaws 20 cannot be moved closer to each other due to the presence of an object having predetermined strength or more, such as, for example, a bone, between the jaws 20, a high load may be applied between the first connection member 310 and the second connection member 320 (between the first convex-concave portion 315 and the second convex-concave portion 317). As such, when a high load is applied between the first connection member 310 and the second connection member 320 (between the first convex-concave portion 315 and the second convex-concave portion 317), as illustrated in FIG. 8, the second connection member 320 may be rotated about the rotating shaft 321. That is, when a high load is applied between the first connection member 310 and the second connection member 320 (between the first convex-concave portion 315 and the second convex-concave portion 317), the force of rotating the second connection member 320 about the rotating shaft 321 becomes greater than the force of pressing the second connection member 320 toward the first connection member 310 by the pressure piece 330, whereby the second connection member 320 may be rotated about the rotating shaft 321. As such, when the second connection member 320 is rotated about the rotating shaft 321, the coupling between the first connection member 310 and the second connection member 320 (between the first convex-concave portion 315 and the second convex-concave portion 317) may be released. Accordingly, the power applied to the handle unit 100 may not be transmitted to the power transmission unit 200, and only the handle unit 100 may be rotated. Because the transmission of power to the power transmission unit 200 is cut off, it is possible to prevent a high load from being applied to, for example, the pressure plate 70, the cam channel 400, and the jaws 20, which are connected to the power transmission unit 200, thereby preventing the breakage thereof.

Meanwhile, the pressure piece 330 for pressing the second connection member 320 toward the first connection member 310 may be, for example, a spring, and may be coupled to a coupling protrusion 335 of the power transmission unit 200 so as to press the other surface of the second connection member 320 (i.e. the surface provided with the second convex-concave portion 317). However, the pressure piece 330 may need to use a spring, and all kinds of pressure pieces known in the art, which are capable of pressing the second connection member 320, may be used.

In addition, the relationship between, for example, a predetermined load at the predetermined position A on the power transmission unit 200, the transmission of power to which is cut off by the power cutoff device 300 (i.e. a load for releasing the coupling between the first convex-concave portion 315 and the second convex-concave portion 317), the predetermined angle of inclination of the first convex-concave portion 315 and the second convex-concave portion 317, the coefficient of friction between the first convex-concave portion 315 and the second convex-concave portion 317, the distance from the main shaft 110 to the position at which the first convex-concave portion 315 and the second convex-concave portion 317 are coupled to each other, the distance from the main shaft 110 to the predetermined position A on the power transmission unit 200, and the force applied to press the second connection member 320 (the second convex-concave portion 317) by the pressure piece 330 will be described below.

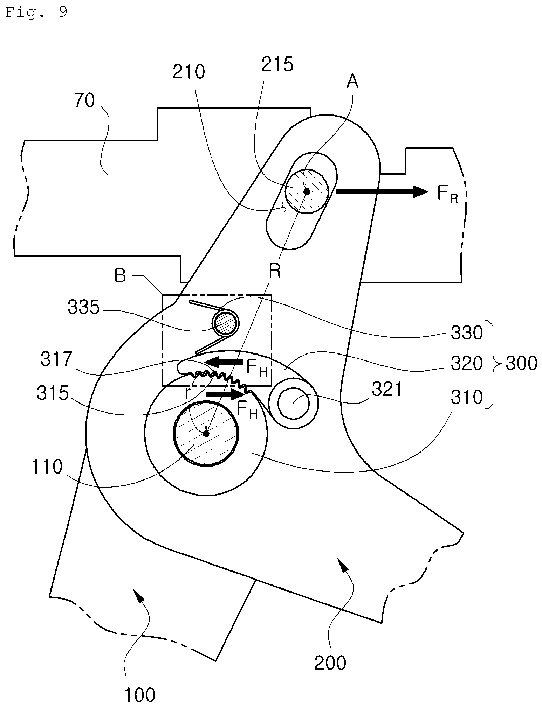

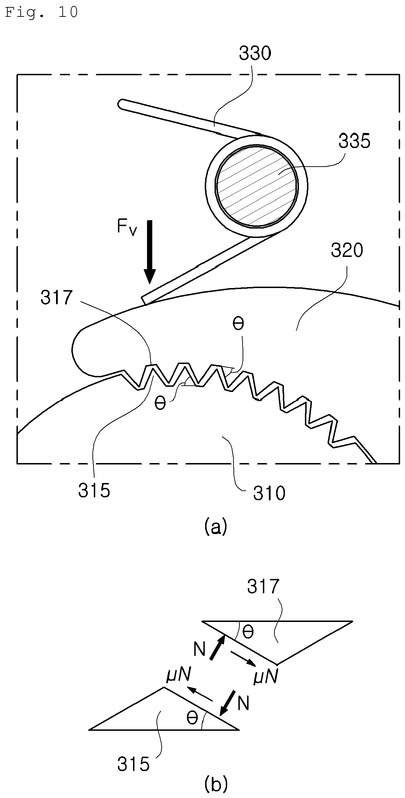

FIGS. 9 and 10 are enlarged views illustrating the power cutoff device of the surgical clip applier according to the embodiment of the present invention.

As illustrated in FIG. 9, a load (repulsive force) that acts on the predetermined position A on the power transmission unit 200, which is connected to the pressure plate 70, may be defined as "$F_R$", a load that acts between the first convex-concave portion 315 and the second convex-concave portion 317 may be defined as "$F_H$", the distance from the main shaft 110 to the position at which the first convex-concave portion 315 and the second convex-concave portion 317 are coupled to each other may be defined as "r", and the distance from the main shaft 110 to the predetermined position A on the power transmission unit 200 may be defined as "R".

At this time, the following relationship is established between $F_H$ and $F_R$.

$$F_R \ X \ R \ = \ F_H \ X \ r$$

$$F_H \ = \ (R/r) \ X \ F_R$$

In addition, as illustrated in FIG. 10, the predetermined angle of inclination of the first convex-concave portion 315 and the second convex-concave portion 317 may be defined as "$\theta$", the normal force applied to the slopes of the first convex-concave portion 315 and the second convex-concave portion 317 may be defined as "N", the coefficient of friction between the first convex-concave portion 315 and the second convex-concave portion 317 may be defined as "$\mu$", and the force applied to press the second connection member 320 (the second convex-concave portion 317) by the pressure piece 330 may be defined as "$F_V$".

At this time, the following relationship is established between $F_V$ and $F_R$.

$$F_H = N \ X \ \sin(\theta) \ + \ \mu \ X \ N \ X \ \cos(\theta)$$

$$F_V = N \ X \ \cos(\theta) \ - \ \mu \ X \ N \ X \ \sin(\theta)$$

$$F_V = [\{\cos(\theta) - \mu \ X \sin(\theta)\}/\{\sin(\theta) + \mu \ X \ \cos(\theta)\}] \ X \ F_H$$

$$F_V = \cot(\theta + \Phi) \ X \ (R/r) \ X \ F_R$$

Here,

$$\mu = \tan(\Phi), \ \Phi = \tan^{-1}(\mu)$$

In the above relational expression, because $F_R$ is the load (repulsive force) that acts on the predetermined position A on the power transmission unit 200 connected to the pressure plate 70, the load may substantially be used as a predetermined load that acts on the predetermined position A on the power transmission unit 20, the transmission of power to which is cut off by the power cutoff device 300.

Therefore, assuming that the predetermined load applied to cut off the power by the power cutoff device 300 is defined as "$F_R$", the predetermined angle of inclination of the first convex-concave portion 315 and the second convex-concave portion 317 is defined as "$\theta$", the coefficient of friction between the first convex-concave portion 315 and the second convex-concave portion 317 is defined as "$\mu$", the distance from the main shaft 110 to the position at which the first convex-concave portion 315 and the second convex-concave portion 317 are coupled to each other is defined as "r", the distance from the main shaft 110 to the predetermined position A on the power transmission unit 200 is defined as "R", and the force with which the pressure piece 330 presses the second connection member 320 (the second convex-concave portion 317) is defined as "$F_V$", the following Equation 1 may be satisfied.

[Equation 1]

$$F_V = \cot(\theta + \Phi) \ \text{X} \ (R/r) \ \text{X} \ F_R, \ \text{here} \ \Phi = \tan^{-1}(\mu)$$

For example, when the predetermined load $F_R$ applied to cut off the power by the power cutoff device 300 is 1000 N, the predetermined angle θ of inclination of the first convex-concave portion 315 and the second convex-concave portion 317 is 70 degrees, the coefficient of friction μ between the first convex-concave portion 315 and the second convex-concave portion 317 is 0.3, and [the distance R from the main shaft 110 to the first slot 210]/[the distance r from the main shaft 110 to the position at which the first convex-concave portion 315 and the second convex-concave portion 317 are coupled to each other] is 3, the process of calculating the force $F_V$ applied to press the second connection member 320 (the second convex-concave portion 317) by the pressure piece 330 is as follows:

$$F_V = \cot(\theta + \Phi) \ \text{X} \ (R/r) \ \text{X} \ F_R$$

$$= \cot(70° \ \text{X} \ (\pi/180°) + \tan^{-1}(0.3)) \ \text{X} \ 3 \ \text{X} \ (1000N)$$

$$= 174N$$

That is, the force $F_V$ with which the pressure piece 330 presses the second connection member 320 (the second convex-concave portion 317) needs to be 174N. For example, when a spring is used as the pressure piece 300, a spring, the initial resistance of which is 174N, needs to be mounted.

Meanwhile, assuming that the coefficient of friction μ between the first convex-concave portion 315 and the second convex-concave portion 317 is 0.3, the calculated result of cot (θ + Φ) depending on the predetermined angle θ of inclination of the first convex-concave portion 315 and the second convex-concave portion 317 is represented in the following Table 1.

[Table 1]

| Angle of Inclination (θ, deg) | Coefficient of Friction (μ) | Cot(θ + Φ) |
|---|---|---|
| 5° | 0.3 | 2.513 |
| 10° | 0.3 | 1.988 |
| 15° | 0.3 | 1.619 |
| 20° | 0.3 | 1.342 |
| 25° | 0.3 | 1.122 |
| 30° | 0.3 | 0.942 |
| 35° | 0.3 | 0.790 |
| 40° | 0.3 | 0.657 |
| 45° | 0.3 | 0.538 |
| 50° | 0.3 | 0.431 |
| 55° | 0.3 | 0.331 |
| 60° | 0.3 | 0.236 |
| 65° | 0.3 | 0.146 |
| 70° | 0.3 | 0.058 |
| 75° | 0.3 | -0.030 |
| 80° | 0.3 | -0.117 |
| 85° | 0.3 | -0.207 |

As described in the above Table 1, when the predetermined angle θ of inclination of the first convex-concave portion 315 and the second convex-concave portion 317 is 75 degrees or more, the value of cot ($\theta$ + $\Phi$) is a negative number. When the value of cot($\theta$ + $\Phi$) is a negative number, consequently, the value of the force $F_V$ with which the pressure piece 330 presses the second connection member 320 is a negative number. This substantially means that the first convex-concave portion 315 and the second convex-concave portion 317 are not separated from each other. Accordingly, the predetermined angle $\theta$ of inclination of the first convex-concave portion 315 and the second convex-concave portion 317 may be 70 degrees or less.

In addition, when the predetermined angle $\theta$ of inclination of the first convex-concave portion 315 and the second convex-concave portion 317 is 55 degrees or less, the cot ($\theta$ + $\Phi$) has a large value of 0.331. When the value of cot($\theta$ + $\Phi$) is large, consequently, the value of the force $F_V$ with which the pressure piece 330 presses the second connection member 320 is excessively increased. Accordingly, the predetermined angle $\theta$ of inclination of the first convex-concave portion 315 and the second convex-concave portion 317 may be 60 degrees or more.

As a result, the predetermined angle $\theta$ of inclination of the first convex-concave portion 315 and the second convex-concave portion 317 may range from 60 degrees to 70 degrees. However, this corresponds to the case where the coefficient of friction $\mu$ is 0.3, of course, the range of the predetermined angle $\theta$ of inclination of the first convex-concave portion 315 and the second convex-concave portion 317 may be changed.

As is apparent from the above description, according to the present invention, through the provision of a power cutoff device that cuts off power when a high load is applied to, for example, a trigger, jaws, or a power system, a surgical clip applier may prevent the breakage of the trigger, the jaws, or the power system. Although the embodiments of the present invention have been described above in detail, it is clear that the above description is merely given to concretely describe the present invention and the present invention is not limited thereto, and that alterations or improvements thereof are possible by those skilled in the art within the scope of the present invention.

The simplified alterations and modifications of the present invention fall within the scope of the present invention, and the concrete protection range of the present invention will be made clear by the accompanying claims.

<Reference numerals>

| | |
|---|---|
| 10: trigger | 20: jaws |
| 25: jaw supports | 30: shaft |
| 40: rotating knob | 50: main body |
| 60: case | 70: pressure plate |
| 75: connection hole | 80: transfer plate |
| 85: third slot | 90: transfer link |
| 95: second slot | |
| 97: opposite end of the transfer link | |
| 100: handle unit | 110: main shaft |
| 200: power transmission unit | |
| 210: first slot | |
| 215: connection pin | 220: pivot body |
| 225: protrusion | 300: power cutoff device |
| 310: first connection member | |
| 315: first convex-concave portion | |
| 320: second connection member | |
| 321: rotating shaft | |
| 317: second convex-concave portion | |
| 330: pressure piece | |
| 335: coupling protrusion | 400: cam channel |
| 410: first coupling | 500: clip track |
| 510: transfer spring | 520: transfer shoe |
| 600: transfer bar | 610: second coupling |
| 620: compression spring | 700: support member |
| A: predetermined position | |
| C: clip | |
| T: blood vessel or the tissues | |

**Claims**

1. A surgical clip applier comprising:

   a handle unit coupled to one side of a case so as to be rotatable about a main shaft;
   a power transmission unit connected to the handle unit for transmitting power to a pair of jaws at a predetermined position thereof so as to move the jaws closer to each other when the handle unit is rotated; and
   a power cutoff device provided between the handle unit and the power transmission unit,

   wherein the power cutoff device includes:

   a first connection member coupled to the handle unit so as to be rotated along with the handle unit about the main shaft;
   a second connection member coupled to the power transmission unit so as to be rotatable about a rotating shaft, which is formed on the power transmission unit; and
   a pressure piece for pressing the second connection member so that one surface of the second connection member is brought into contact with and is coupled with one surface of the first connection member,

   wherein, in a state in which the surface of the first connection member is brought into contact with and is coupled with the surface of the second connection member, when the first connection member is rotated about the main shaft, the second connection member is rotated, along with the first connection member, about the main shaft, and wherein, when a predetermined load or more is applied to the predetermined position on the power transmission unit, the second connection member is rotated about the rotating shaft, and coupling between the first connection member and the second connection member is released.

2. The applier according to claim 1, wherein the surface of the first connection member is provided with a first convex-concave portion in which convex portions and concave portions are successively formed,
   wherein the surface of the second connection member is provided with a second convex-concave portion in which convex portions and concave portions are successively formed, and
   wherein the first convex-concave portion and the second convex-concave portion are engaged with and coupled to each other.

3. The applier according to claim 2, wherein the first convex-concave portion is provided on the surface of the first connection member that extends and has a predetermined curvature, and
   wherein the second convex-concave portion is provided on the surface of the second connection member that extends and has a predetermined curvature, so as to correspond to the first convex-concave portion.

4. The applier according to claim 3, wherein the first connection member has a cylindrical shape so that the first convex-concave portion is formed in a predetermined section of an outer circumferential surface thereof, and
   wherein the second connection member extends to have an arc shape so that the second convex-concave portion is formed in a predetermined section of a side surface thereof, which faces the first convex-concave portion.

5. The applier according to claim 2, wherein the first convex-concave portion is formed in a shape of teeth so as to have a predetermined angle of inclination,
   wherein the second convex-concave portion is formed in a shape of teeth so as to have a predetermined angle of inclination, and
   wherein the predetermined angle of inclination of the first convex-concave portion and the predetermined angle of inclination of the second convex-concave portion are the same as each other.

6. The applier according to claim 1, wherein the pressure piece is a spring.

7. The applier according to claim 5, wherein, when the predetermined load is defined as "$F_R$", the predetermined angle of inclination of the first convex-concave portion and the second convex-concave portion is defined as "$\theta$ ", a coefficient of friction between the first convex-concave portion and the second convex-concave portion is defined as "$\mu$ ", a distance from the main shaft to a position at which the first convex-concave portion and the second convex-concave portion are coupled to each other is defined as "r", a distance from the main shaft to the predetermined position on the power transmission unit is defined as "R", and force with which the pressure piece presses the second connection member is defined as "$F_V$", the following Equation 1 is satisfied.

[Equation 1]

$$F_V = \cot(\theta + \Phi) \ X \ (R/r) \ X \ F_R, \ here \ \Phi = \tan^{-1}(\mu)$$

8.  The applier according to claim 5, wherein the predetermined angle of inclination of the first convex-concave portion and the second convex-concave portion ranges from 60 degrees to 70 degrees.

9.  The applier according to claim 1, wherein the power transmission unit is connected at the predetermined position to a pressure plate, and
    wherein, when a user rotates the handle unit, the power transmission unit is rotated about the main shaft, causing the pressure plate to slide.

10. The applier according to claim 9, wherein the pressure plate is connected to a cam channel, and
    wherein, when the pressure plate slides, the cam channel slides toward the jaws, causing a pair of jaw support portions for supporting the jaws to be moved closer to each other inside the cam channel, and consequently, causing the jaws to be moved closer to each other.

Fig. 1

Fig. 2

Fig. 3

Fig. 4

Fig. 5

Fig. 6A

Fig. 6B

Fig. 6C

Fig. 6D

Fig. 7

Fig. 8

Fig. 9

Fig. 10

(a)

(b)

Europäisches
Patentamt

European
Patent Office

Office européen
des brevets

**EUROPEAN SEARCH REPORT**

Application Number

EP 16 19 8798

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 908 426 A1 (TYCO HEALTHCARE [US]) 9 April 2008 (2008-04-09) * paragraphs [0042] - [0043], [0050] - [0065]; figures 3, 8-12 * | 1-10 | INV. A61B17/10 A61B17/128 A61B17/12 |
| A | US 5 782 834 A (LUCEY PAUL V [US] ET AL) 21 July 1998 (1998-07-21) * the whole document * | 1 | ADD. A61B17/00 A61B17/29 A61B90/00 |
| A | US 2012/004684 A1 (REINAUER JOSEF [DE]) 5 January 2012 (2012-01-05) * paragraphs [0034], [0044] - [0053]; figures 1-5 * | 1-3,6-9 | |
| A | US 2008/255608 A1 (HINMAN CAMERON D [US] ET AL) 16 October 2008 (2008-10-16) * the whole document * | 1-3,6,9, 10 | |

TECHNICAL FIELDS
SEARCHED       (IPC)

A61B

The present search report has been drawn up for all claims

| Place of search | Date of completion of the search | Examiner |
|---|---|---|
| Munich | 7 July 2017 | Kink, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 19 8798

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2017

| Patent document cited in search report | | | Publication date | Patent family member(s) | | | Publication date |
|---|---|---|---|---|---|---|---|
| EP | 1908426 | A1 | 09-04-2008 | AU | 2007219294 | A1 | 24-04-2008 |
| | | | | CA | 2603911 | A1 | 05-04-2008 |
| | | | | EP | 1908426 | A1 | 09-04-2008 |
| | | | | ES | 2336616 | T3 | 14-04-2010 |
| | | | | JP | 5184034 | B2 | 17-04-2013 |
| | | | | JP | 2008093437 | A | 24-04-2008 |
| | | | | JP | 2013027722 | A | 07-02-2013 |
| | | | | US | 2008083813 | A1 | 10-04-2008 |
| | | | | US | 2014150600 | A1 | 05-06-2014 |
| US | 5782834 | A | 21-07-1998 | AT | 164992 | T | 15-05-1998 |
| | | | | AU | 679196 | B2 | 26-06-1997 |
| | | | | CA | 2114330 | A1 | 30-07-1994 |
| | | | | DE | 69409565 | D1 | 20-05-1998 |
| | | | | DE | 69409565 | T2 | 01-10-1998 |
| | | | | EP | 0613661 | A2 | 07-09-1994 |
| | | | | JP | H06269460 | A | 27-09-1994 |
| | | | | US | 5620415 | A | 15-04-1997 |
| | | | | US | 5782834 | A | 21-07-1998 |
| US | 2012004684 | A1 | 05-01-2012 | DE | 102009031262 | A1 | 05-01-2011 |
| | | | | EP | 2269522 | A1 | 05-01-2011 |
| | | | | US | 2012004684 | A1 | 05-01-2012 |
| US | 2008255608 | A1 | 16-10-2008 | US | 2008255608 | A1 | 16-10-2008 |
| | | | | US | 2013218140 | A1 | 22-08-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101298521 B1